# EUROPEAN PATENT APPLICATION

(11) **EP 1 978 021 A1**
(43) Date of publication of application: **08.10.2008**
(21) Application number: 07105578.4
(22) Date of filing: 03.04.2007
(51) Int. Cl.: C07D 257/04, A61K 31/41, A61P 31/04

(54) **Novel antibiotics**

(71) Applicant: Nederlandse Organisatie voor toegepast- natuurwetenschappelijk onderzoek TNO, 2628 VK The Hague (NL)
(72) Inventor: Schuren, Frank Henri Johan, 3902 EB Veenendaal (NL); Montijn, Roy Christiaan, 1028 BJ Amsterdam (NL)
(74) Representative: van Loon, C.J.J.

(57) **Abstract**

The invention relates to the field of antibiotic compositions, both inside and outside the medical field. Presented is a new class of antibiotic compounds, variants of bis(1-aryl-5-tetrazolyl)methanes, which are especially useful for combating infections with gram-positive bacteria and especially MRSA.

## Description

The invention relates to the field of pharmaceutical compounds, especially antibiotic compounds.

Searching for novel antibiotic compounds has become more and more important, especially since many micro-organisms are becoming resistant to known antibiotic compounds. This is especially the case for a group of *Staphylococcus aureus* bacteria, which are now identified as MRSA (methicillin-resistant *S. aureus*). Therefore, there is an ever increasing need for new antibiotic compounds, which can be used against micro-organisms that have become resistant to commonly used antibiotics.

The present inventors have developed a new test and detection system to search for novel antibiotics and novel targets for antibiotics. This system is the subject of several copending applications (WO 03/0087397, WO 03/0981389, WO 05/0035782, WO 05/106033). Using this system now a new class of antibiotics has been uncovered, which have been described in PCT/NL2006/000489. During the experiments with this new class of antibiotics further variants were discovered, which displayed antibiotic activity

The presently disclosed new variants cover compounds with the general formula (I): 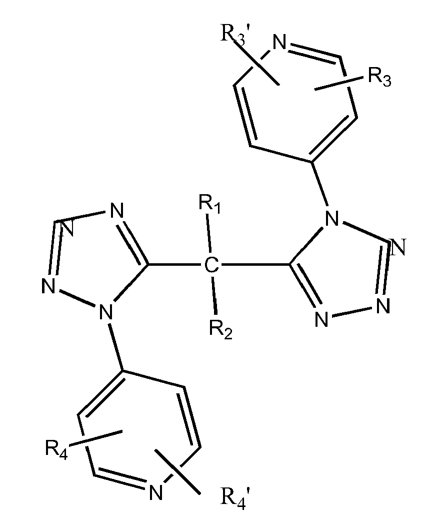 wherein R₁ and R₂ are each independently halogen, lower alkyl or absent, with the proviso that R₁ and R₂ are not both absent, and wherein R₃, R₃', R₄ and R₄' are each independently absent, OH, SO₂NH₃, lower alkyl, lower alkenyl, lower alkoxy, lower alkoxy (methyl), aryl, heteroaryl, wherein the alkyl, alkoxy, aryl and heteroaryl may be substituted, arylalkoxy or halogen.

Further, said variants can be of the general formula (II) 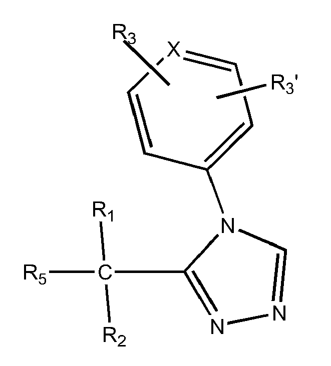 wherein X = C, or N, R₁, R₂, R₃ and R₃, are the same as in Formula (I) and R₅ can be chosen from the group consisting of hydrogen, hydroxy, halogen, trifluoromethyl, trichloromethyl, tribromomethyl, C(O)OR₆,OC(O)R₆, C(O)NR₆, NC(O)R₆, C(O)SR₆, S(O)OR₆, PO₃R₆, HPO₂R₆, H₂POR₆, lower alkenyl, lower alkynyl, lower alkoxy, lower alkoxy (methyl), nitro, formyl, lower alkoxy carbonyl, mercapto, lower alkylthio, and lower alkyldithio, aromatic electron donating groups such as furyl, thienyl, pyrazolyl, pyrrolyl, imidazolyl, indolyl, thiazolyl, oxazolyl, isothiszolyl, isoxazolyl, piperidyl, pyrrolinyl, piperazinyl, quinolyl, triazolyl, tetrazolyl, isoquinolyl, benozofuryl, benzothienyl, morpholinyl, benzoxazolyl, tetrahydrofuryl, pyranyl, indazolyl, purinyl, indolinyl, pyrazolindinyl, imidazolinyl, imidazolindinyl, pyrrolidinyl, furazanyl, N-methylindolyl, methylfuryl, pyridazinyl, pyrimidinyl, pyrazinyl, epoxy, aziridino, oxetanyl or azetidinyl, or any electron donating group, with the proviso that said aromatic electron donating group is not tetrazolophenyl or tetrazolo substituted phenyl, and wherein R₆ is hydrogen, hydroxy, SO₂NH₃, lower alkyl, lower alkenyl, lower alkoxy, lower alkoxy (methyl), aryl, heteroaryl, wherein the alkyl, alkoxy, aryl and heteroaryl may be substituted, arylalkoxy or halogen.

The term "alkyl" or "lower alkyl" refers to a straight or branched alkyl radical containing one to six carbon atoms including, but not limited to, methyl, ethyl, propyl, isopropyl, n-butyl, t-butyl and neopentyl. Similarly, the term lower alkenyl and lower alkynyl refer to C₁₋₆alkenyl and C₁₋₆alkynyl, respectively.

The term "loweralkoxy" refers to an alkyl or loweralkyl group as previously defined, i.e. with one to six C atoms, attached to a parent molecular moiety by an ether linkage.

The term "loweralkoxy (methyl)" refers to an alkoxy group as described above attached to a parent molecular moiety via a methylene group (-CH₂-).

The term "aryl" as used herein refers to a mono-or bicyclic carbocyclic ring system having one or two aromatic rings including, but not limited to, phenyl, naphthyl, tetrahydronaphthyl, indanyl, indenyl and the like. Aryl groups (including bicyclic aryl groups) can be unsubstituted or substituted with one, two or three substituents independently selected from loweralkyl, substituted loweralkyl, haloalkyl, alkoxy, thioalkoxy, amino, alkylamino, dialkylamino, acylamino, benzyloxycarbonyl, cyano, hydroxyl, halo, mercapto, nitro, carboxaldehyde, carboxy, alkoxycarbonyl, carboxamide, and protected hydroxyl. In addition, substituted aryl groups include tetrafluorophenyl and pentafluorophenyl.

The term "heteroaryl", as used herein, refers to a mono-or bicyclic fused aromatic radical having from five to ten ring atoms of which one ring atom is selected from S, O and N; zero, one or two ring atoms are additional heteroatoms independently selected from S, O and N; and the remaining ring atoms are carbon, the radical being joined to the rest of the molecule via any of the ring atoms, such as, for example, pyridyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl, isoquinolinyl, and the like.

The term "substituted alkyl or alkoxy" as used herein refers to an alkyl or alkoxy group as defined herein substituted by independent replacement of one, two or three of the hydrogen atoms thereon with Cl, Br, F, I, OH, CN, haloalkyl, thioalkoxy, amino, alkylamino, dialkylamino, mercapto, nitro, carboxaldehyde, carboxy, alkoxycarbonyl and carboxamide.

The term "substituted aryl" as used herein refers to an aryl group as defined herein substituted by independent replacement of one, two or three of the hydrogen atoms thereon with Cl, Br, F, I, OH, CN, C₁-C₃-alkyl, C₁-C₆-alkoxy, C₁-C₆-alkoxy substituted with aryl, haloalkyl, thioalkoxy, amino, alkylamino, dialkylamino, mercapto, nitro, carboxaldehyde, carboxy, alkoxycarbonyl and carboxamide. In addition, any one substituent may be an aryl, heteroaryl, or heterocycloalkyl group. Also, substituted aryl groups include tetrafluorophenyl and pentafluorophenyl.

The term "substituted heteroaryl" as used herein refers to a heteroaryl group as defined herein substituted by independent replacement of one, two or three of the hydrogen atoms thereon with Cl, Br, F, I, OH, CN, C₁-C₃-alkyl, C₁-C₆-alkoxy, C₁-C₆-alkoxy substituted with aryl, haloalkyl, thioalkoxy, amino, alkylamino, dialkylamino, mercapto, nitro, carboxaldehyde, carboxy, alkoxycarbonyl and carboxamide. In addition, any one substituent may be an aryl, heteroaryl, or heterocycloalkyl group.

The term "pharmaceutically acceptable salts" as used herein refers to those carboxylate salts, esters, and prodrugs of the compound of the present invention which are, within the scope of sound medical judgment, suitable for use in contact with the tissues of humans and lower animals with undue toxicity, irritation, allergic response, and the like, commensurate with a reasonable benefit/risk ratio, and effective for their intended use, as well as the zwitterionic forms, where possible, of the compounds of the invention.

Pharmaceutically acceptable salts are well known in the art and refer to the relatively non-toxic, inorganic and organic acid addition salts of the compounds of the present invention. For example, S. M. Berge, et al. describe pharmaceutically acceptable salts in detail in J. Pharmaceutical Sciences, 66: 1-19 (1977) which is incorporated herein by reference. The salts can be prepared in situ during the final isolation and purification of the compounds of the invention, or separately by reacting the free base function with a suitable organic acid.

Examples of pharmaceutically acceptable, non-toxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxyethanesulfonate, lactobionate, lactate, laurate, lauryl sulfate, malate, maleate, malonate, methanesulfonate, 2-naphthalenesulfonate, nicotinate, nitrate, oleate, oxalate, palmitate, palmoate, pectinate, persulfate, 3-phenylpropionate, phosphate, picrate, pivalate, propionate, stearate, succinate, sulphate, tartrate, thiocyanate, p-toluenesulphonate, undecanoate, valerate salts, and the like. Representative alkali or alkaline earth metal salts include sodium, lithium, potassium, calcium, magnesium, and the like. Further pharmaceutically acceptable salts include, when appropriate, non-toxic ammonium, quaternary ammonium, and amine cations formed using counterions such as halide, hydroxide, carboxylate. sulphate, phosphate, nitrate, loweralkyl sulphonate and aryl sulphonate.

As used herein, the term "pharmaceutically acceptable ester" refers to esters which hydrolyze in vivo and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Examples of particular esters include formates, acetates, propionates, butyrates, acrylates and ethylsuccinates.

The term "pharmaceutically acceptable solvate" represents an aggregate that comprises one or more molecules of the solute, such as a compound of the invention, with one or more molecules of solvent.

The term "pharmaceutically acceptable prodrugs" as used herein refers to those prodrugs of the compounds of the present invention which are, within the scope of sound medical judgment, suitable for use in contact with the tissues of humans and lower animals without undue toxicity, irritation, allergic response, and the like, commensurate with a reasonable benefit/risk ratio, and effective for their intended use, as well as the zwitterionic forms, where possible, of the compounds of the invention. The term "prodrug" refers to compounds that are rapidly transformed in vivo to yield the parent compound of the above formula, for example by hydrolysis in blood. A thorough discussion is provided in T. Higuchi and V. Stella, Pro-drugs as Novel Delivery Svstems, Vol. 14 of the A. C. S. Symposium Series, and in Edward B. Roche, ed., Bioreversible Carriers in Drug Design, American Pharmaceutical Association and Pergamon Press, 1987, both of which are incorporated herein by reference.

Preferred compounds according to formula (I) are those compounds in which one or both of R₁ and R₂ is Cl. Also preferred are compounds according to formula (I) and (II) wherein R₃ and R₄ are located at the ortho position of the phenyl moieties and R₃' and R₄' are absent. Most preferred is a compound of formula (I), wherein R₁=R₂=Cl, R₃=R₄=CH₃ and R₃' and R₄' are absent and wherein R₃ and R₄ are in the ortho position.

### Preferred compounds of formula (II) are compounds

Especially preferred are N1-phenyl-2,2-dichloro-2-(1-phenyl-1H-1,2,3,4-tetraazol-5-yl)acetamide (NewCo0052) and *N*1-(2-methoxyphenyl)-2,2-dichloro-2-(1-phenyl-1H-1,2,3,4-tetraazol-5-yl)acetamide (NewCo0053).

In general, a synthetic route for (several of) the compounds as disclosed in PCT/NL2006/000489 has been presented by Shivanyuk, A.F. et al., (1986) Zhurnal Organicheskoi Chimii 22(1):200-205, while the electronic structure of these molecules has been provided in Pen'kovskii et al., (1991) Zhurnal Organicheskoi Chimii 27(1):148-153. In short (see Scheme 1), synthesis of these compounds starts from malonic acid di-esters (ROC(O)CC(O)OR) which are converted, in the present case with aniline or derivatives thereof, to the corresponding diamide (RNHC(O)CC(O)NHR) which are subsequently reacted with PCl₅ to a chlorinated imidoyl chloride derivative. After reaction with sodium azide the tetrazole derivatives are isolated

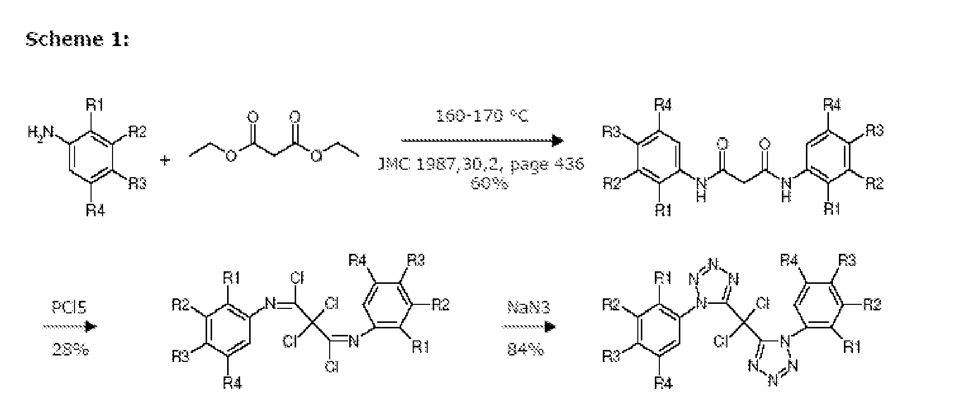

The amidation reaction has been described by Vennerstrom, J.L. and Holmes, T.J. Jr., (1987) J. Med. Chem. 30: 434-437). This scheme, but with 4-aminopyridine in stead of aniline as starting compound, can be used to produce the compounds of Formula (I).

Asymmetric compounds of formula (I), i.e. where the R-groups at the phenyl rings are not identical can be made by reacting two different 4-aminopyridine derivatives with the malonic acid di-esters (Scheme 2):

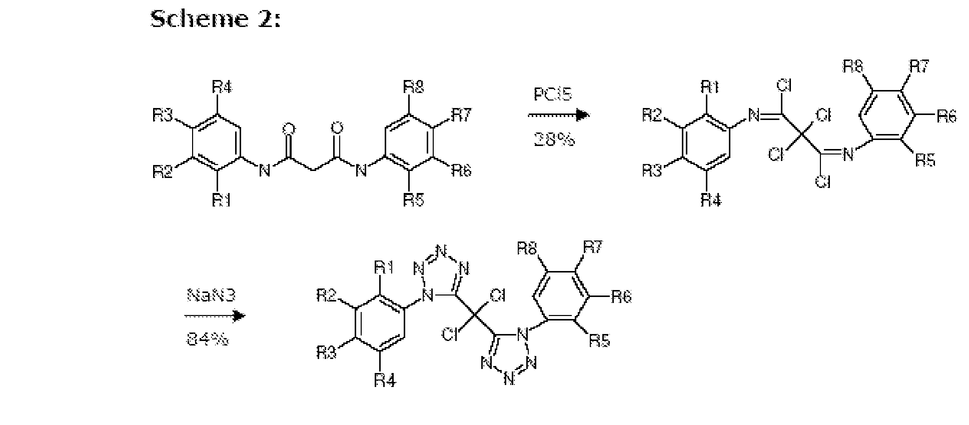

A general scheme for the production of the monotetrazole compounds of Formula (II) is given below:

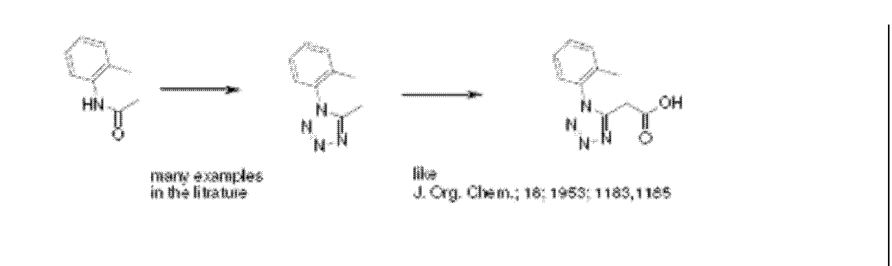

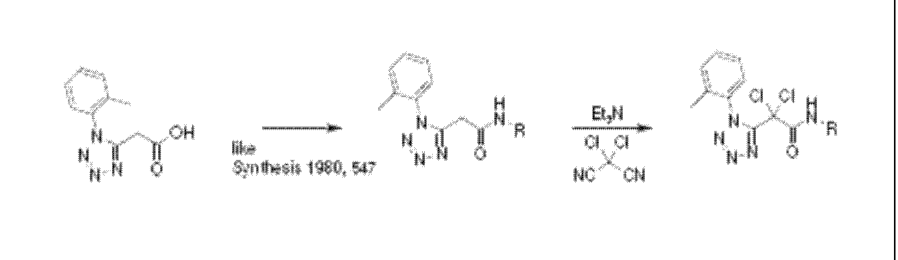

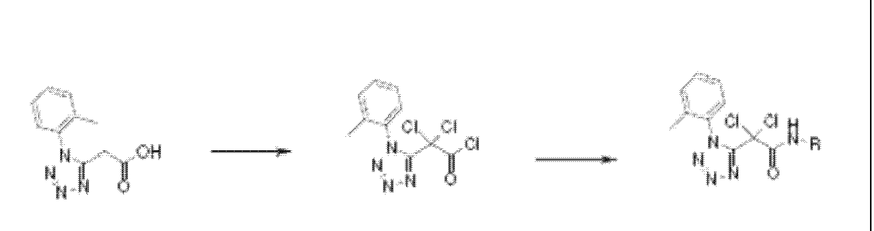

The compounds of Formula (II) are especially attractive because at one side the amide bond can be used to attach various moieties. One of the possibilities is to couple hydrophilic moieties to the molecule to increase the solubility of the compound. A preferred alternative is to provide the molecule with a moiety which itself has a biologic activity. This activity can be an antibiotic activity, such that the total spectrum of antibiotic activity of the complete molecule is broadened. Especially preferred are antibiotics which work against Gram negative bacteria, such as beta-lactams, like penicillin, ampicillin, amoxycillin, oxacillin, carbenicillin and derivatives, ticarcillin, aziocillin, methycillin, mezocillin, and piperacillin, cephalosporins such as cephapirin, cefazolin, cephalexin, cefadroxil, cephradine, cefamandolenafate, cefonicid, cefuroxime, cefoxitim, cefotetan, cefaclor, cefprozil, loracarbacef, cefotaxime, ceftazidime, ceftozoxime, cefoperazone, ceftriaxone, cefixime, ceftibutem, cefdinir and cefpodoxime proxetil, carbapenems such as imipenem and miripinem, aminoglycosides such as gentamycin, amikacin and tobramycin, tazobactam, clindamycin, ciprofloxacin, sulbactam, aztrianam, chloramphenicol, colistin, nitrofurantoin, tetracycline, polymixin B, neomycin, trimethoprim and sulfamethoxazole.

The moiety which can attached to the compounds of formula (II) can also be a moiety which is able to target the molecule to a specific locus. A typical example of such a moiety would be an antibody or antibody fragment which is able to bind to an epitope which is present at a certain location (i.e.the surface of a bacterial cell).

The compounds according to formula (I) and (II) have antibiotic activity, in particular against Gram positive bacteria. They are especially active against staphylococcal and enterococcal strains, and in particular against *S. aureus,* including also the strains of *S. aureus,* that are commonly known as MRSA strains.

They can be used in pharmaceutical compositions for the treatment of bacterial diseases, especially those diseases caused by the above mentioned micro-organisms, or in conditions wherein the subject runs the risk of being infected with micro-organisms.

The compounds of the invention or compositions therewith can, however, also be used in other than pharmaceutical applications, e.g. in cosmetics (e.g. for the treatment of acne), in detergents and/or other cleaning solutions, in anti-fouling paints, in food or feed or in food or feed packaging, and so on.

A compound according to the formula (I) or (II), or a pharmaceutically acceptable salt or prodrug thereof, may be provided to a subject in need thereof for prophylactic or therapeutic reasons. A compound according to the formula (I) or (II), or a pharmaceutically acceptable salt or prodrug thereof, may be provided to a subject in need thereof in the form of any pharmaceutical preparation, when such administration form is capable of treating and/or preventing infection in a subject. As a consequence of the prevention or treatment of infection, also the clinical effects or sequellae of infection will be prevented.

The present invention also relates to a method for preventing and/or treating infection in a subject, preferably a human or other mammalian subject, said method comprising administering to said subject a therapeutically and/or prophylactically effective amount of a pharmaceutical composition comprising a compound according to formula (I) or (II), r pharmaceutically acceptable salts or prodrugs thereof and a pharmaceutically acceptable carrier, and optionally one or more excipients.

The present invention also relates to the use of a compound according to formula (I) or (II), or pharmaceutically acceptable salts or prodrugs thereof for the manufacture of a medicament for treating infection, preferably bacterial infections, most preferably staphylococcal or enterococcal infection.

An antibiotic therapy (i.e. the method for preventing and/or treating infection in a subject) may also comprise administering to an otherwise healthy individual, at risk of developing infection, a prophylactically effective amount of the pharmaceutical composition.

Dosages for achieving the antibiotic effects of the pharmaceutical composition described herein may easily be determined by the skilled person. For purposes of the present invention, an effective dose will be a daily dose between about 0.01 mg and 10 grams of the compound according to formula (I) for an adult human being. More preferably a dose between 0,1 mg and 1 gram is used, even more preferably a dose of 1 mg - 100 mg and most preferably a dose of 4-40 mg of the compound of the invention is administered. This daily dose may be given as a one-dose administration, or it may be subdivided in several subdoses, which are administered spread over the day.

For oral administration, the compositions may be packed in e.g. gelatin capsules or may be tableted in the form of tablets. For oral therapeutic application the active compound may be administered with excipients and e.g. used in the form of powders, sachets, tablets, pills, pastilles or capsules. The pharmaceutical compositions may be prepared by conventional means with pharmaceutically acceptable excipients such as binding agents (e.g. pregelatinised maize starch, tragacanth gum, gelatine, polyvinylpyrrolidone or hydroxypropyl methylcellulose); fillers (e.g. lactose, microcrystalline cellulose, mannitol or calcium hydrogen phosphate); lubricants (e.g. magnesium stearate, talc or silica); disintegrants (e.g. potato starch, sodium starch glycollate or alginate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or acacia); nonaqueous vehicles (e.g. almond oil, oily esters, ethyl alcohol or fractionated vegetable oils); and preservatives (e.g. methyl or propyl-p-hydroxybenzoates or sorbic acid). The preparations may also contain buffer salts, flavoring, coloring and sweetening agents as appropriate. Preparations for oral administration may be suitably formulated to give controlled release of the active compound.

For buccal administration the compositions may take the form of tablets or lozenges formulated in conventional manner.

The compounds according to the present invention may be formulated for parenteral administration by injection e.g. by bolus injection or continuous infusion. Formulations for injection may be presented in unit dosage form e.g. in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g. sterile pyrogen-free water, before use.

When dosing is in the form of a capsule, the capsule may comprise apart from the elements mentioned above a liquid carrier such as an oil. Dosage form may further be provided with coatings of sugar, shellac or other agents. The components of the pharmaceutical composition are preferably chosen such that they do not reduce the desired working of the active compound.

The pharmaceutical compositions can further comprise flavoring sweetening, coloring and/or preservative agents.

A compound according to the formula (I) or (II), or a pharmaceutically acceptable salt or prodrug thereof may also be administered in the form of e.g. an elixir, a suspension, a syrup, a waffle or a chewing gum.

In a pharmaceutical composition as described above, a compound according to the formula (I) or (II), or a pharmaceutically acceptable salt or prodrug thereof, is used in an amount of from 0.01 to 99.9 % by weight, preferably from 0.01 to 10 wt.%, and more preferably from 0.05 to 5 wt.%.

The present invention further relates to a method for the preparation of a pharmaceutical composition for preventing and/or treating infection, comprising processing or incorporating a compound according to the formula (I) or (II), or a pharmaceutically acceptable salt or prodrug thereof, as an active substance, together with a pharmaceutically acceptable carrier in a pharmaceutical composition.

The preparation of a pharmaceutical composition may very suitably occur by mixing all separate ingredients such as fillers, binders, lubricants and optionally other excipients together with a compound according to the formula (I) or (II), or a pharmaceutically acceptable salt or prodrug thereof, and processing the mixture obtained to a pharmaceutical preparation.

### EXAMPLES

### Example 1. Synthesis of NewCo0052

### N1-phenyl-3-anilino-2,2-dichloro-3-oxopropanimidoyl chloride

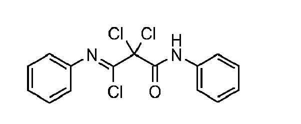

At room temperature, phosphorus pentachloride (284 mg, 1.37 mmol) was added to a solution of *N*1,*N*3-diphenylmalonamide (79 mg, 0.311 mmol) in xylene (10 mL). The reaction was warmed at reflux for 3 hours. Phosphorus pentachloride (64 mg, 0.311 mmol) was added and the reaction was continued for 1.5 hours. The reaction was cooled to room temperature and the solvent was evaporated to dryness in vacuo. The residue was co-evaporated with toluene and then the product was dried at 140 °C and high vacuum (1 x 10⁻² torr) for 0.5 hours to afford *N*1-phenyl-3-anilino-2,2-dichloro-3-oxopropanimidoyl chloride. The crude product was used as such in the preparation of *N*1-phenyl-2,2-dichloro-2-(1-phenyl-1*H*-1,2,3,4-tetraazol-5-yl)acetamide.

NB: *N*1,*N*3-diphenylmalonamide was purchased from a commercial supplier (Enamine).

### N1-phenyl-2,2-dichloro-2-(1-phenyl-1H-1,2,3,4-tetraazol-5-yl)acetamide

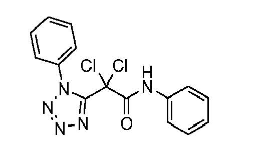

At 0 °C, sodium azide (202 mg, 3.11 mmol) was added to a solution of *N*1-phenyl-3-anilino-2,2-dichloro-3-oxopropanimidoyl chloride (0.311 mmol) in acetone (10 mL). The reaction was stirred at room temperature for 72 hours and then at reflux for 3 hours. The reaction was cooled to room temperature and a saturated aqueous solution of sodium chloride (25 mL) was added. The aqueous mixture was extracted with ethyl acetate (2 x 40 mL). The combined organic extracts were washed with saturated aqueous sodium bicarbonate (25 mL) and saturated aqueous sodium chloride (25 mL). The organic layer was dried (Na₂SO₄), filtered and evaporated to dryness in vacuo. The crude product was purified by flash column chromatography (ethyl acetate/heptane, from 15% to 50% ethyl acetate). The solvent was co-evaporated three times with dichloromethane to afford 31.1 mg (29%) of *N*1-phenyl-2,2-dichloro-2-(1-phenyl-1*H*-1, 2, 3, 4-tetraazol-5-yl)acetamide.

1H-NMR (CDCl₃, 400 MHz) δ 7.20-7.25 (m, 1H), 7.34-7.43 (m, 4H), 7.47-7.57 (m, 5H), 8.60 (bs, 1H).

### Example 2. Synthesis of NewCo0053

### N1-(2-methoxyphenyl)-2,2-dichloro-3-(2-methoxyanilino)-3-oxopropanimidoyl chloride

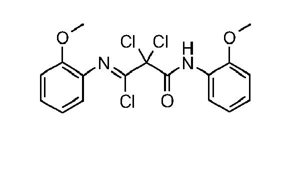

At room temperature, phosphorus pentachloride (146 mg, 0.700 mmol) was added to a solution of *N*1,*N*3-di(2-methoxyphenyl)malonamide (50 mg, 0.159 mmol) in toluene (5 mL). The reaction was warmed at reflux for 1 hour. The reaction was cooled to room temperature and diluted with toluene (5 mL). The reaction was washed with water (5 mL), saturated aqueous sodium bicarbonate (5 mL) and saturated aqueous sodium chloride (5 mL). The organic layer was diluted with ethyl acetate, dried (Na₂SO₄), filtered, and evaporated to dryness in vacuo to afford *N*1-(2-methoxyphenyl)-2,2-dichloro-3-(2-methoxyanilino)-3-oxopropanimidoyl chloride as a brown/yellow oil in a yield of 77 mg (120%). The crude product was used as such for the preparation of N1-(2-methoxyphenyl)-2,2-dichloro-2- [1-(2-methoxyphenyl)-1*H*-1,2,3,4-tetraazol-5-yl]acetamide.

NB: *N*1,*N*3-di(2-methoxyphenyl)malonamide) was purchased from a commercial supplier (Enamine).

### N1-(2-methoxyphenyl)-2,2-dichloro-2-[1-(2-methoxyphenyl)-1H-1,2,3,4-tetraazol-5-yl]acetamide

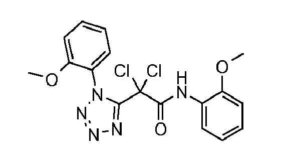

At 0 °C, sodium azide (119 mg, 1.83 mmol) was added to a solution of *N*1-(2-methoxyphenyl)-2,2-dichloro-3-(2-methoxyanilino)-3-oxopropanimidoyl chloride (0.183 mmol) in acetone (5 mL). The reaction was stirred at room temperature for 18 hours. The reaction was cooled to room temperature and a saturated aqueous solution of sodium chloride (25 mL) was added. The aqueous mixture was extracted with ethyl acetate (2 x 40 mL). The combined organic extracts were washed saturated aqueous sodium chloride (25 mL). The organic layer was dried (Na₂SO₄), filtered and evaporated to dryness in vacuo. The crude product was purified by flash column chromatography (ethyl acetate/heptane, from 15% to 35% ethyl acetate). The solvent was co-evaporated three times with dichloromethane to afford 18.8 mg (25%) of *N*1-(2-methoxyphenyl)-2,2-dichloro-2-[1-(2-methoxyphenyl)-1*H*-1,2,3,4-tetraazol-5-yl] acetamide

1H-NMR (CDCl₃, 400 MHz) δ 3.66 (s, 3H), 3.89 (s, 3H), 6.88-7.02 (m, 4H), 7.14 (dt, 1H, *J =* 1.5 Hz, J *=* 8.1 Hz), 7.40 (dd, 1H, *J =* 1.5 Hz, J = 7.8 Hz), 7.46 (dt, 1H, *J*= 1.8 Hz, J= 8.1 Hz), 8.18 (dd, 1H, J= 1.5 Hz, *J*= 8.1 Hz), 9.15 (bs, 1H).

### EXAMPLE 2 MIC Tests

MIC test were performed according to standard methodology: M7-A6-"Methods for Dilution Antimicrobial Susceptibility Tests for Bacteria That Grow Aerobically"; *Approved Standard,* Sixth Edition Clinical and Laboratory Standards Institute 2005 (CLSI/formerly NCCLS).

Table I lists the results of 2 different compounds according to Formula (II), i.e. the compounds designated as NEWCo0052 and NewCo0053 on single strains of *S. aureus* and *S. epidermis* as MIC values expressed in micrograms per milliliter.

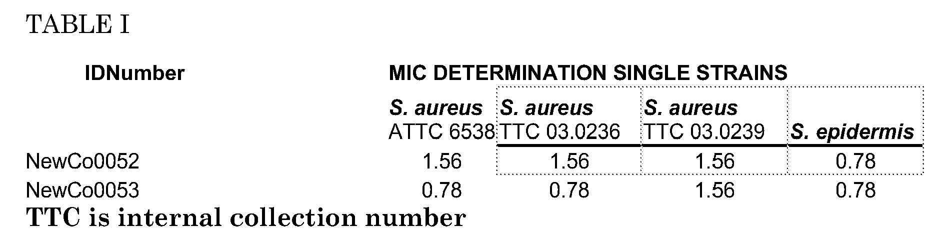

## Claims

1. A compound according to formula (I) 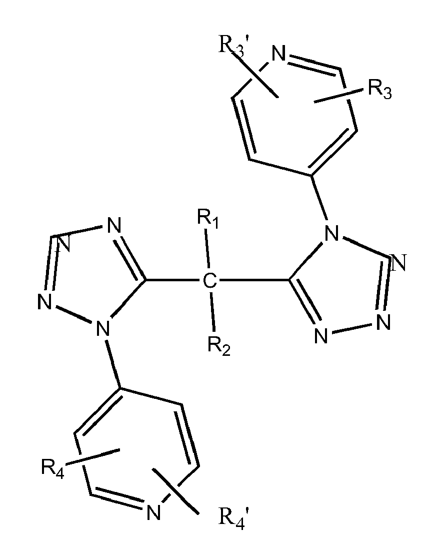 wherein R1 and R2 are each independently halogen, lower alkyl or absent, with the proviso that R1 and R2 are not both absent, and wherein R3, R3', R4 and R4' are each independently absent, OH, SO₂NH₃, lower alkyl, lower alkoxy, lower alkoxy (methyl), aryl, heteroaryl, wherein lower alky, aryl and heteroaryl may be substituted, arylalkoxy or halogen.

2. A compound according to formula (II): 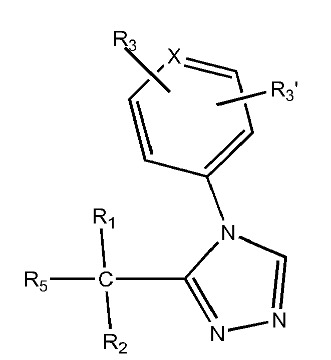 wherein X = C, or N, R₁, R₂, R₃ and R₃, are the same as in Formula (I) and R₅ can be chosen from the group consisting of hydrogen, hydroxy, halogen, trifluoromethyl, trichloromethyl, tribromomethyl, C(O)OR₆,OC(O)R₆, C(O)NR₆, NC(O)R₆, C(O)SR₆, S(O)OR₆, PO₃R₆, HPO₂R₆, H₂POR₆, lower alkenyl, lower alkynyl, lower alkoxy, lower alkoxy (methyl), nitro, formyl, lower alkoxy carbonyl, mercapto, lower alkylthio, and lower alkyldithio, aromatic electron donating groups such as furyl, thienyl, pyrazolyl, pyrrolyl, imidazolyl, indolyl, thiazolyl, oxazolyl, isothiszolyl, isoxazolyl, piperidyl, pyrrolinyl, piperazinyl, quinolyl, triazolyl, tetrazolyl, isoquinolyl, benozofuryl, benzothienyl, morpholinyl, benzoxazolyl, tetrahydrofuryl, pyranyl, indazolyl, purinyl, indolinyl, pyrazolindinyl, imidazolinyl, imidazolindinyl, pyrrolidinyl, furazanyl, N-methylindolyl, methylfuryl, pyridazinyl, pyrimidinyl, pyrazinyl, epoxy, aziridino, oxetanyl or azetidinyl, or any electron donating group, with the proviso that said aromatic electron donating group is not tetrazolophenyl or tetrazolo substituted phenyl, and wherein R₆ is hydrogen, hydroxy, SO₂NH₃, lower alkyl, lower alkenyl, lower alkoxy, lower alkoxy (methyl), aryl, heteroaryl, wherein the alkyl, alkoxy, aryl and heteroaryl may be substituted, arylalkoxy or halogen.

3. A compound according to claim 1 or 2, wherein either R₁ or R₂ or both are Cl.

4. A compound according to any of claims 1-3, wherein R₃' and R₄' are absent and R3 or R₄ or both are linked to the phenyl moiety at the ortho position.

5. A compound according to any of the above mentioned claims, wherein R₃ and/or R₄ are selected from the group comprising CH₃, Cl, or O-CH₃.

6. A compound selected from the group consisting essentially of: *N*1-phenyl-2,2-dichloro-2-(1-phenyl-1*H*-1,2,3,4-tetraazol-5-yl)acetamide and *N*1-(2-methoxyphenyl)-2,2-dichloro-2-(1-phenyl-1*H-*1,2,3,4-tetraazol-5-yl)acetamide.

7. A compound according to any of claims 1-6, or a pharmaceutically acceptable salt, prodrug, ester or solvate thereof, for use as a medicament.

8. A compound according to any of claims 1-6, or a pharmaceutically acceptable salt, prodrug, ester or solvate thereof, for use as antibiotic.

9. A pharmaceutical composition comprising a compound according to any of claims 1-6 and a pharmaceutically acceptable carrier.

10. Use of a compound according to any of claims 1-6 or a pharmaceutically acceptable salt, prodrug, ester or solvate thereof for the manufacture of a medicament for the treatment of bacterial infection.
